# EUROPEAN PATENT APPLICATION

(11) **EP 4 813 967 A1**
(43) Date of publication of application: **30.09.2026**
(21) Application number: 25849631.4
(22) Date of filing: 17.12.2025
(51) Int. Cl.: C07C 43/12

(54) **OXAHYDROFLUOROETHER COMPOUND, AND SYNTHESIS METHOD THEREFOR AND USE THEREOF**

(30) Priority: 06.02.2025 CN 202510133835
(71) Applicant: Zhejiang Noah Fluorochemical Co., Ltd., Shaoxing, Zhejiang 312300 (CN); Hubei Noah New Material Science & Technology Co., Ltd., Shengzhixiaxianjixingzhengquhua, Hubei 433100 (CN)
(72) Inventor: ZHANG, Miaomiao, Zhejiang 312300 (CN); ZHANG, Peng, Zhejiang 312300 (CN); SONG, Weichang, Zhejiang 312300 (CN); SHI, Lei, Zhejiang 312300 (CN); LIU, Liang, Zhejiang 312300 (CN)
(74) Representative: Kicinska-Fujawa, Alicja
(86) International application number: PCT/CN2025/143255
(87) International publication number: WO 2026/166192

(57) **Abstract**

The present application belongs to the technical field of synthesis of fluorine-containing fine chemicals and specifically relates to an oxa-hydrofluoroether compound and a synthesis method and a use thereof. An oxa-hydrofluoroether compound, having a structure as shown in Formula (1): 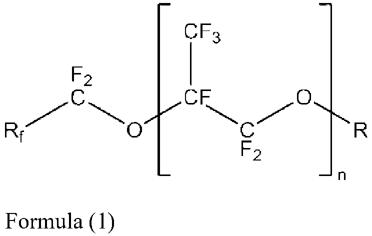 wherein, R_{f} is F or CF₃; R is methyl or ethyl; n is an integer of 1-3. For the oxa-hydrofluoroether compound disclosed in the present application, due to the embedding of oxygen atoms in the fluorocarbon chain structure, the fluorine-containing segments of hydrofluoroether are more diversified, such that the structure is no longer limited to a perfluorocarbon chain structure. In such an oxa-hydrofluoroether compound, the oxygen atoms play a role in reducing the surface tension, and the more oxygen atoms the oxa-hydrofluoroether compound contains, the lower the surface tension. Compared with other hydrofluoroether solvents, the oxa-hydrofluoroether compound exhibits stronger permeability and better cleaning performance. Due to the embedding of oxygen atoms into the fluorocarbon chain structure and the presence of branched fluorocarbon segments, the oxa-hydrofluoroether compound is more readily degradable, thereby improving environmental friendliness.

## Description

### Technical Field

The present application belongs to the technical field of synthesis of fluorine-containing fine chemicals and specifically relates to an oxa-hydrofluoroether compound and a synthesis method and a use thereof.

### BACKGROUND ART

With the rapid development of information technologies such as artificial intelligence, cloud computing, big data, and blockchain, the requirements for processing performance and integration of data center servers and communication equipment are getting higher and higher, leading to a continuous increase in power density. In recent years, there has been a high growth trend in the demand for environmentally friendly electronic cleaning agents and fluorinated electronic coolants in this field.

Hydrofluoroethers are a class of ether compounds containing fluorine, carbon, hydrogen, and oxygen, belonging to partially fluorinated ethers. They are a new generation of substitutes for ozone-depleting substances and have a series of excellent properties such as low viscosity, low freezing point, low surface tension, and good electrochemical stability. Different from chlorofluorocarbons, hydrofluoroether compounds do not contain other halogens other than fluorine and basically do not affect the Earth's ozone layer. Therefore, hydrofluoroether compounds exhibit an ozone depletion potential of zero. In addition, hydrofluoroether compounds are more readily degraded in the Earth's atmosphere and have a relatively lower global warming potential (GWP), making them ideal substitutes for chlorofluorocarbon compounds. Hydrofluoroethers have unparalleled advantages over other substitutes for ozone-depleting substances in the fields of electronics industry, cleaning agents for precision instruments, lubricant diluents, polymerization solvents, leak detection fluids, heat transfer media, electronic coolants, medical anesthetics, etc.

Therefore, the development of new, green and environmentally friendly hydrofluoroether compounds has become an urgent problem to be solved.

### SUMMARY

To address the deficiencies of the prior art, the present application provides an oxa-hydrofluoroether compound and a synthesis method and a use thereof.

The oxa-hydrofluoroether compound disclosed in the present application only contains fluorine as the halogen element and does not contain substances that damage the ozone layer. Accordingly, it is an environmentally friendly compound.

In a first aspect, the present application provides an oxa-hydrofluoroether compound, adopting the following technical solution:

An oxa-hydrofluoroether compound, having a structure as shown in Formula (1): 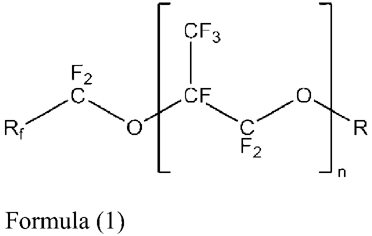 wherein, R_{f} is F or CF₃; R is methyl or ethyl; and n is an integer of 1-3.

Preferably, the oxa-hydrofluoroether compound is any one of:

CF₃OCF(CF₃)CF₂OCH₃;

CF₃OCF(CF₃)CF₂OCF(CF₃)CF₂OCH₃;

CF₃OCF(CF₃)CF₂OCF(CF₃)CF₂OCF(CF₃)CF₂OCH₃;

CF₃OCF(CF₃)CF₂OCH₂CH₃;

CF₃OCF(CF₃)CF₂OCF(CF₃)CF₂OCH₂CH₃;

CF₃OCF(CF₃)CF₂OCF(CF₃)CF₂OCF(CF₃)CF₂OCH₂CH₃;

CF₃CF₂OCF(CF₃)CF₂OCH₃;

CF₃CF₂OCF(CF₃)CF₂OCF(CF₃)CF₂OCH₃;

CF₃CF₂OCF(CF₃)CF₂OCF(CF₃)CF₂OCF(CF₃)CF₂OCH₃;

CF₃CF₂OCF(CF₃)CF₂OCH₂CH₃;

CF₃CF₂OCF(CF₃)CF₂OCF(CF₃)CF₂OCH₂CH₃;

or

CF₃CF₂OCF(CF₃)CF₂OCF(CF₃)CF₂OCF(CF₃)CF₂OCH₂CH₃.

Further preferably, the oxa-hydrofluoroether compound is any one of:

CF₃OCF(CF₃)CF₂OCH₃;

CF₃OCF(CF₃)CF₂OCF(CF₃)CF₂OCH₃;

CF₃CF₂OCF(CF₃)CF₂OCH₃;

CF₃CF₂OCF(CF₃)CF₂OCF(CF₃)CF₂OCH₃;

CF₃OCF(CF₃)CF₂OCH₂CH₃;

CF₃OCF(CF₃)CF₂OCF(CF₃)CF₂OCH₂CH₃;

CF₃CF₂OCF(CF₃)CF₂OCH₂CH₃;

or

CF₃CF₂OCF(CF₃)CF₂OCF(CF₃)CF₂OCH₂CH₃.

More preferably, the oxa-hydrofluoroether compound is selected from:

CF₃OCF(CF₃)CF₂OCH₃ or CF₃CF₂OCF(CF₃)CF₂OCH₂CH₃.

In a second aspect, the present application provides a synthesis method of an oxa-hydrofluoroether compound, adopting the following technical solution:

A synthesis method of an oxa-hydrofluoroether compound includes the following steps: reacting an acyl fluoride compound, an alkylating reagent and an alkaline metal fluoride in a polar aprotic solvent to obtain the oxa-hydrofluoroether compound.

Preferably, the acyl fluoride compound has a structure as shown in Formula (2): 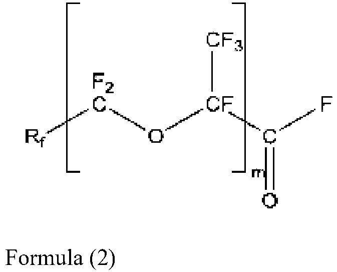 wherein, R_{f} is F or CF₃; and m is an integer of 1-3.

Preferably, the acyl fluoride compound is one or more of:

CF₃OCF(CF₃)COF;

CF₃OCF(CF₃)CF₂OCF(CF₃)COF;

CF₃OCF(CF₃)CF₂OCF(CF₃)CF₂OCF(CF₃)COF;

CF₃CF₂OCF(CF₃)COF;

CF₃CF₂OCF(CF₃)CF₂OCF(CF₃)COF;

or

CF₃CF₂OCF(CF₃)CF₂OCF(CF₃)CF₂OCF(CF₃)COF.

Further preferably, the acyl fluoride compound is selected from:

CF₃OCF(CF₃)COF or CF₃CF₂OCF(CF₃)COF.

Preferably, the alkylating reagent is at least one of dialkyl sulfate, dialkyl carbonate, or halogenated hydrocarbon.

Further preferably, the alkylating reagent is the dialkyl sulfate.

Further preferably, the dialkyl sulfate is dimethyl sulfate or diethyl sulfate.

Preferably, the alkaline metal fluoride is at least one of potassium fluoride, sodium fluoride, cesium fluoride, or rubidium fluoride.

Preferably, the polar aprotic solvent is at least one of ethylene glycol dimethyl ether, ethylene glycol dibutyl ether, diethylene glycol dimethyl ether, diethylene glycol dibutyl ether, tetraethylene glycol monomethyl ether, tetraethylene glycol dimethyl ether, acetonitrile, N,N-dimethylformamide, N,N-diethylformamide, N-methylpyrrolidone, or dimethyl sulfoxide.

Preferably, a molar ratio of the acyl fluoride compound to the alkylating reagent is 1:(1-1.5).

Further preferably, the molar ratio of the acyl fluoride compound to the alkylating reagent is 1:(1-1.2).

Preferably, a molar ratio of the alkaline metal fluoride to the acyl fluoride compound is 1:(2-10).

Further preferably, the molar ratio of the alkaline metal fluoride to the acyl fluoride compound is 1:(4-8).

Preferably, a mass ratio of the acyl fluoride compound to the polar aprotic solvent is 1:(0.8-2).

Further preferably, the mass ratio of the acyl fluoride compound to the polar aprotic solvent is 1:(0.8-1.8).

Preferably, the synthesis method of an oxa-hydrofluoroether compound includes the following steps:
(1) under a protection of nitrogen, mixing the alkaline metal fluoride with the polar aprotic solvent;
(2) after cooling, under the protection of nitrogen, adding an acyl fluoride compound for mixing to obtain a mixture;
(3) dropwise adding an alkylating reagent into the mixture obtained in the step (2), and heating to react after dropwise addition is completed;
(4) after cooling, adding the alkaline liquor for mixing, followed by standing and distillation to obtain the oxa-hydrofluoroether compound with a purity ≥ 99.5%.

Preferably, a temperature after cooling in the step (2) is 0-20°C.

Preferably, before cooling in the step (2), a reaction pressure is adjusted to 0.01-1.0 MPa.

Preferably, the temperature after heating in the step (3) is 30-120°C, and a reaction time is 2-24 h.

Preferably, the temperature after cooling in the step (4) is 20-30°C.

Preferably, the alkaline liquor in the step (4) is at least one of a sodium hydroxide solution, a potassium hydroxide solution, a sodium carbonate solution, or a potassium carbonate solution; and a mass concentration of the alkaline liquor is 5-50%.

Further preferably, the alkaline liquor is the sodium hydroxide solution, and the mass concentration of the alkaline liquor is 10-20%.

In one specific feasible embodiment, the synthesis method of an oxa-hydrofluoroether compound specifically includes the following operation steps:
S-1: under the protection of nitrogen, adding an anhydrous alkaline metal fluoride into a stainless steel high-pressure reactor;
S-2: after evacuating and purging the reactor, continuing to add a dried polar aprotic solvent to obtain a mixture; adjusting a reaction pressure of the reactor to 0.01-1.0 MPa, while stirring to uniformly mix materials and simultaneously cooling the reactor;
S-3: after cooling the reactor to 0-20°C, under the protection of nitrogen, adding the acyl fluoride compound into the mixture obtained in S-2 for mixing;
S-4: then dropwise adding the alkylating reagent into the mixture obtained in the step S-3 within 0.5 h through a dropping funnel;
S-5: then heating to 30-120°C for reaction and continuing the reaction for 2-24 h to complete the reaction;
S-6: after cooling the reactor to 20-30°C, transferring a resulting mixture obtained in the step S-5 into a flask, adding the alkaline liquor, continuing stirring for a period of time, then standing for phase separation;
S-7: taking a lower fluorine phase for distillation to obtain the oxa-hydrofluoroether compound with a purity ≥ 99.5%.

In a third aspect, the present application provides a use of the oxa-hydrofluoroether compound in a preparation of an electronic cleaning agent, an electronic fluorinating liquid, or a solvent, adopting the following technical solution:

A use of the oxa-hydrofluoroether compound of the first aspect or the oxa-hydrofluoroether compound prepared by the method of the second aspect in a preparation of an electronic cleaning agent, an electronic fluorinating liquid, or a solvent.

The series of oxa-hydrofluoroether compounds provided by the present application can be applied in various fields: they may be used as electronic cleaning agents in fields such as semiconductor, liquid crystal, and hard disk manufacturing; as electronic fluorinating liquids for cooling and temperature control in various stages of semiconductor manufacturing (such as manufacturing cooling machines, temperature control of upper and lower electrodes in dry etching processes, exposure tools for chip patterning, high- and low-temperature cooling control in chip testing systems, chip sorting equipment, etc.) as well as cooling and heat dissipation in the heat dissipation systems of heat-generating components in military radar systems and wind turbine generators; and as solvents in applications such as electronic coatings and screen layer diluents.

In summary, the present application includes at least one of the following beneficial technical effects:
1. For the oxa-hydrofluoroether compound disclosed in the present application, due to the embedding of oxygen atoms in the fluorocarbon chain structure, the fluorine-containing segments of hydrofluoroether are more diversified, such that the structure is no longer limited to a perfluorocarbon chain structure.
2. For the oxa-hydrofluoroether compound disclosed in the present application, the oxygen atoms play a role in reducing the surface tension, and the more oxygen atoms the oxa-hydrofluoroether compound contains, the lower the surface tension. Compared with other hydrofluoroether solvents, the oxa-hydrofluoroether compound exhibits stronger permeability and better cleaning performance.
3. Due to the embedding of oxygen atoms into the fluorocarbon chain structure and the presence of branched fluorocarbon segments, the oxa-hydrofluoroether compound disclosed in the present application is more readily degradable, thereby improving environmental friendliness.

### Brief Description of the Drawings

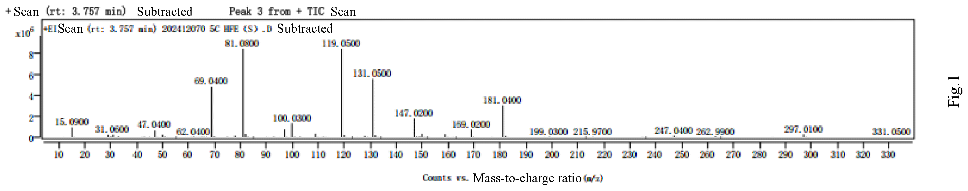

FIG. 1 shows a GC-MS spectrum of CF₃CF₂OCF(CF₃)CF₂OCH₃ prepared in Example 2.

### Detailed Description

In the following examples of the present application, unless otherwise specified, experimental methods are generally carried out under conventional conditions or according to the conditions recommended by the manufacturer. The experimental materials and reagents involved in the following content are, unless otherwise specified, commercially available products.

Unless otherwise defined, all technical and scientific terms used in the present application have the same meanings as commonly understood by those skilled in the technical field to which the present application pertains. The terminology used in the specification of the present application is solely for describing specific embodiments and is not intended to limit the present application.

The terms "comprising" and "having" and any variations thereof in the present application are intended to cover non-exclusive inclusion. For example, a process, method, device, product, or equipment that includes a series of steps is not limited to the listed steps or modules, but may optionally also include steps not listed, or other inherent steps of these processes, methods, products, or equipment.

The technical solutions of the present application will be clearly and completely described below with reference to specific examples. It is apparent that the described examples are only part of the examples of the present application, rather than all of them. All other examples obtained by those with ordinary skills in the art based on the examples of the present application without creative efforts shall fall within the scope of protection of the present application.

### Source of raw materials:

1. Preparation of acyl fluoride compounds of CF₃OCF(CF₃)COF, CF₃OCF(CF₃)CF₂OCF(CF₃)COF and CF₃OCF(CF₃)CF₂OCF(CF₃)CF₂OCF(CF₃)COF:
   First, a catalyst of tetramethylethylenediamine (20.4 g) and an aprotic polar solvent of triethylene glycol dimethyl ether (498.5 g) were successively introduced into a clean and dry 2-L sealed stainless steel high-pressure reactor. Then the reactor was cooled while stirring at a speed of 200 rpm. When a temperature of the reactor dropped to -15°C, carbonyl fluoride (50 g) was introduced into the reactor and then stirred continually for 10 min. Subsequently, hexafluoropropylene oxide (132.4 g) was introduced into the reactor at a rate of 0.1 g/min for reaction and then stirred continually for 1 h to complete the reaction to obtain a reaction solution. The reaction solution was transferred into a separatory funnel and left standing for phase separation. An upper layer was an organic phase, and a lower layer was a fluorine phase. Samples were taken for chromatographic analysis, showing that the fluorine phase was a mixture of CF₃OCF(CF₃)COF, CF₃OCF(CF₃)CF₂OCF(CF₃)COF, and CF₃OCF(CF₃)CF₂OCF(CF₃)CF₂OCF(CF₃)COF with contents of 57.3%, 36.8%, and 3.91% respectively.

The fluorine phase was taken for rectification to obtain CF₃OCF(CF₃)COF (89.3 g), CF₃OCF(CF₃)CF₂OCF(CF₃)COF (57.4 g), and CF₃OCF(CF₃)CF₂OCF(CF₃)CF₂OCF(CF₃)COF (6.0 g) respectively, with a purity ≥ 99%.

2. Preparation of acyl fluoride compounds of CF₃CF₂OCF(CF₃)COF, CF₃CF₂OCF(CF₃)CF₂OCF(CF₃)COF and CF₃CF₂OCF(CF₃)CF₂OCF(CF₃)CF₂OCF(CF₃)COF:
First, a catalyst of tetramethylethylenediamine (20.4 g) and an aprotic polar solvent of triethylene glycol dimethyl ether (498.5 g) were successively introduced into a clean and dry 2-L sealed stainless steel high-pressure reactor. Then the reactor was cooled while stirring at a speed of 200 rpm. When a temperature of the reactor dropped to -15°C, trifluoroacetyl fluoride (125.8 g) was introduced into the reactor and then stirred continually for 10 min. Subsequently, hexafluoropropylene oxide (132.4 g) was introduced into the reactor at a rate of 0.2 g/min for reaction and then stirred continually for 1 h to complete the reaction to obtain a reaction solution. The reaction solution was transferred into a separatory funnel and left standing for phase separation. An upper layer was an organic phase, and a lower layer was a fluorine phase. Samples were taken for chromatographic analysis, showing that the fluorine phase was a mixture of CF₃CF₂OCF(CF₃)COF, CF₃CF₂OCF(CF₃)CF₂OCF(CF₃)COF and CF₃CF₂OCF(CF₃)CF₂OCF(CF₃)CF₂OCF(CF₃)COF with contents of 30.2%, 52.6%, 15.1% respectively.

The fluorine phase was taken for rectification to obtain CF₃CF₂OCF(CF₃)COF (66.3 g), CF₃CF₂OCF(CF₃)CF₂OCF(CF₃)COF (115.4 g) and CF₃CF₂OCF(CF₃)CF₂OCF(CF₃)CF₂OCF(CF₃)COF (33.1 g) respectively, with a purity ≥ 99%.

### Example 1:

### Preparation of CF₃OCF(CF₃)CF₂OCH₃:

Into a 2-L jacketed stainless steel high-pressure reactor equipped with a stirrer, a pressure gauge, and a feeding funnel, under the protection of nitrogen, potassium fluoride (25.00 g, 0.43 mol) was added, and the reactor was then sealed. Subsequently, the reactor was evacuated and purged three times. Under the protection of nitrogen, a dried diethylene glycol dimethyl ether (800 g) was added to obtain a first mixture, and the reactor was depressurized to 0.01 MPa. While stirring at a speed of 300 rpm, the reactor was simultaneously cooled to 0°C. Under the protection of nitrogen, CF₃OCF(CF₃)COF (500 g, 2.16 mol) was continuously added into the first mixture. Then, dimethyl sulfate (325.42 g, 2.58 mol) was dropwise added within 0.5 h. The temperature of the reactor was raised to 60°C for reaction, and a resulting mixture was continuously stirred for 8 h to complete the reaction to obtain a reaction solution. After cooling the reactor to room temperature (25°C), the reaction solution was transferred into a flask, a sodium hydroxide aqueous solution (340 g) with a mass concentration of 10% was then added and stirred for 10 min to obtain a second mixture. Then, the stirring was stopped and the second mixture was left standing for 0.5 h. A lower fluorine phase was released for distillation to obtain a target product CF₃OCF(CF₃)CF₂OCH₃.

### Example 2:

### Preparation of CF₃CF₂OCF(CF₃)CF₂OCH₃:

Into a 2-L jacketed stainless steel high-pressure reactor equipped with a stirrer, a pressure gauge, and a feeding funnel, under the protection of nitrogen, potassium fluoride (29.05 g, 0.50 mol) was added, and the reactor was then sealed. Subsequently, the reactor was evacuated and purged three times. Under the protection of nitrogen, a dried tetraethylene glycol dimethyl ether (900 g) was added to obtain a first mixture, and the reactor was depressurized to 0.01 MPa. While stirring at a speed of 300 rpm, the reactor was simultaneously cooled to 10°C. Under the protection of nitrogen, CF₃CF₂OCF(CF₃)COF (557.84 g, 2.12 mol) was continuously added into the first mixture. Then, dimethyl sulfate (315.33 g, 2.5 mol) was dropwise added within 0.5 h. The temperature of the reactor was raised to 90°C for reaction, and a resulting mixture was continuously stirred for 8 h to complete the reaction to obtain a reaction solution. After cooling the reactor to room temperature (25°C), the reaction solution was transferred into a flask, a sodium hydroxide aqueous solution (320 g) with a mass concentration of 10% was then added and stirred for 10 min to obtain a second mixture. Then, the stirring was stopped and the second mixture was left standing for 0.5 h. A lower fluorine phase was released for distillation to obtain a target product CF₃CF₂OCF(CF₃)CF₂OCH₃. The structure of CF₃CF₂OCF(CF₃)CF₂OCH₃ was confirmed by GC-MS, as shown in FIG. 1.

### Example 3:

### Preparation of CF₃OCF(CF₃)CF₂OCF(CF₃)CF₂OCH₃:

Into a 2-L jacketed stainless steel high-pressure reactor equipped with a stirrer, a pressure gauge, and a feeding funnel, under the protection of nitrogen, cesium fluoride (65.32 g, 0.43 mol) was added, and the reactor was then sealed. Subsequently, the reactor was evacuated and purged three times. Under the protection of nitrogen, a dried diethylene glycol dimethyl ether (800 g) was added to obtain a first mixture, and the reactor was depressurized to 0.01 MPa. While stirring at a speed of 300 rpm, the reactor was simultaneously cooled to 20°C. Under the protection of nitrogen, CF₃OCF(CF₃)CF₂OCF(CF₃)COF (655.7 g, 2.15 mol) was continuously added into the first mixture. Then, dimethyl sulfate (325.42 g, 2.58 mol) was dropwise added within 0.5 h. The temperature of the reactor was raised to 90°C for reaction, and a resulting mixture was continuously stirred for 8 h to complete the reaction to obtain a reaction solution. After cooling the reactor to room temperature (25°C), the reaction solution was transferred into a flask, a sodium hydroxide aqueous solution (350 g) with a mass concentration of 10% was then added and stirred for 10 min to obtain a second mixture. Then, the stirring was stopped and the second mixture was left standing for 0.5 h. A lower fluorine phase was released for distillation to obtain a target product CF₃OCF(CF₃)CF₂OCF(CF₃)CF₂OCH₃.

### Example 4:

### Preparation of CF₃CF₂OCF(CF₃)CF₂OCF(CF₃)CF₂OCH₃:

Into a 2-L jacketed stainless steel high-pressure reactor equipped with a stirrer, a pressure gauge, and a feeding funnel, under the protection of nitrogen, cesium fluoride (65.32 g, 0.43 mol) was added, and the reactor was then sealed. Subsequently, the reactor was evacuated and purged three times. Under the protection of nitrogen, a dried diethylene glycol dimethyl ether (800 g) was added to obtain a first mixture, and the reactor was depressurized to 0.01 MPa. While stirring at a speed of 300 rpm, the reactor was simultaneously cooled to 20°C. Under the protection of nitrogen, CF₃CF₂OCF(CF₃)CF₂OCF(CF₃)COF (963.2 g, 2.15 mol) was continuously added into the mixture. Then, dimethyl sulfate (325.42 g, 2.58 mol) was dropwise added within 0.5 h. The temperature of the reactor was raised to 90°C for reaction, and a resulting mixture was continuously stirred for 8 h to complete the reaction to obtain a reaction solution. After cooling the reactor to room temperature (25°C), the reaction solution was transferred into a flask, a sodium hydroxide aqueous solution (350 g) with a mass concentration of 10% was then added and stirred for 10 min to obtain a second mixture. Then, the stirring was stopped and the second mixture was left standing for 0.5 h. A lower fluorine phase was released for distillation to obtain a target product CF₃CF₂OCF(CF₃)CF₂OCF(CF₃)CF₂OCH₃.

### Example 5:

### Preparation of CF₃OCF(CF₃)CF₂OCH₂CH₃:

Into a 2-L jacketed stainless steel high-pressure reactor equipped with a stirrer, a pressure gauge, and a feeding funnel, under the protection of nitrogen, potassium fluoride (25 g, 0.43 mol) was added, and the reactor was then sealed. Subsequently, the reactor was evacuated and purged three times. Under the protection of nitrogen, a dried N,N-dimethylformamide (900 g) was added to obtain a first mixture, and the reactor was depressurized to 0.01 MPa. While stirring at a speed of 300 rpm, the reactor was simultaneously cooled to 0°C. Under the protection of nitrogen, CF₃OCF(CF₃)COF (500 g, 2.16 mol) was continuously added into the first mixture. Then, diethyl sulfate (397.81 g, 2.58 mol) was dropwise added within 0.5 h. The temperature of the reactor was raised to 60°C for reaction, and a resulting mixture was continuously stirred for 8 h to complete the reaction to obtain a reaction solution. After cooling the reactor to room temperature (25°C), the reaction solution was transferred into a flask, a sodium hydroxide aqueous solution (230 g) with a mass concentration of 15% was then added and stirred for 10 min to obtain a second mixture. Then, the stirring was stopped and the second mixture was left standing for 0.5 h. A lower fluorine phase was released for distillation to obtain a target product CF₃OCF(CF₃)CF₂OCH₂CH₃.

### Example 6:

### Preparation of CF₃OCF(CF₃)CF₂OCF(CF₃)CF₂OCH₂CH₃:

Into a 2-L jacketed stainless steel high-pressure reactor equipped with a stirrer, a pressure gauge, and a feeding funnel, under the protection of nitrogen, potassium fluoride (17.43 g, 0.3 mol) was added, and the reactor was then sealed. Subsequently, the reactor was evacuated and purged three times. Under the protection of nitrogen, a dried N,N-dimethylformamide (1000 g) was added to obtain a first mixture, and the reactor was depressurized to 0.01 MPa. While stirring at a speed of 300 rpm, the reactor was simultaneously cooled to 0°C. Under the protection of nitrogen, CF₃OCF(CF₃)CF₂OCF(CF₃)COF (796 g, 2 mol) was continuously added into the first mixture. Then, diethyl sulfate (320.72 g, 2.08 mol) was dropwise added within 0.5 h. The temperature of the reactor was raised to 90°C for reaction, and a resulting mixture was continuously stirred for 10 h to complete the reaction to obtain a reaction solution. After cooling the reactor to room temperature (25°C), the reaction solution was transferred into a flask, a sodium hydroxide aqueous solution (70 g) with a mass concentration of 10% was then added and stirred for 10 min to obtain a second mixture. Then, the stirring was stopped and the second mixture was left standing for 0.5 h. A lower fluorine phase was released for distillation to obtain a target product CF₃OCF(CF₃)CF₂OCF(CF₃)CF₂OCH₂CH₃.

### Example 7:

### Preparation of CF₃CF₂OCF(CF₃)CF₂OCH₂CH₃:

Into a 2-L jacketed stainless steel high-pressure reactor equipped with a stirrer, a pressure gauge, and a feeding funnel, under the protection of nitrogen, potassium fluoride (25 g, 0.43 mol) was added, and the reactor was then sealed. Subsequently, the reactor was evacuated and purged three times. Under the protection of nitrogen, a dried N,N-dimethylformamide (900 g) was added to obtain a first mixture, and the reactor was depressurized to 0.01 MPa. While stirring at a speed of 300 rpm, the reactor was simultaneously cooled to 0°C. Under the protection of nitrogen, CF₃CF₂OCF(CF₃)COF (564 g, 2 mol) was continuously added into the first mixture. Then, diethyl sulfate (331.51 g, 2.15 mol) was dropwise added within 0.5 h. The temperature of the reactor was raised to 80°C for reaction, and a resulting mixture was continuously stirred for 6 h to complete the reaction to obtain a reaction solution. After cooling the reactor to room temperature (25°C), the reaction solution was transferred into a flask, a sodium hydroxide aqueous solution (120 g) with a mass concentration of 10% was then added and stirred for 10 min to obtain a second mixture. Then, the stirring was stopped and the second mixture was left standing for 0.5 h. A lower fluorine phase was released for distillation to obtain a target product CF₃CF₂OCF(CF₃)CF₂OCH₂CH₃.

### Example 8:

### Preparation of CF₃CF₂OCF(CF₃)CF₂OCF(CF₃)CF₂OCH₂CH₃:

Into a 2-L jacketed stainless steel high-pressure reactor equipped with a stirrer, a pressure gauge, and a feeding funnel, under the protection of nitrogen, cesium fluoride (65.32 g, 0.43 mol) was added, and the reactor was then sealed. Subsequently, the reactor was evacuated and purged three times. Under the protection of nitrogen, a dried diethylene glycol dimethyl ether (800 g) was added to obtain a first mixture, and the reactor was depressurized to 0.01 MPa. While stirring at a speed of 300 rpm, the reactor was simultaneously cooled to 20°C. Under the protection of nitrogen, CF₃CF₂OCF(CF₃)CF₂OCF(CF₃)COF (896 g, 2 mol) was continuously added into the first mixture. Then, diethyl sulfate (333.03 g, 2.16 mol) was dropwise added within 0.5 h. The temperature of the reactor was raised to 90°C for reaction, and a resulting mixture was stirred for 10 h to complete the reaction to obtain a reaction solution. After cooling the reactor to room temperature (25°C), the reaction solution was transferred into a flask, a sodium hydroxide aqueous solution (90 g) with a mass concentration of 15% was then added and stirred for 10 min to obtain a second mixture. Then, the stirring was stopped and the second mixture was left standing for 0.5 h. A lower fluorine phase was released for distillation to obtain a target product CF₃CF₂OCF(CF₃)CF₂OCF(CF₃)CF₂OCH₂CH₃.

The reaction results of Examples 1-8 are shown in Table 1.

**Table 1 Reaction results of Examples 1-8**

| Example | Acyl fluoride compound | Dialkyl sulfate | Target product | Yield /% | Purity /% |
|---|---|---|---|---|---|
| 1 | CF₃OCF(CF₃)COF | (CH₃O)₂SO₂ | CF₃OCF(CF₃)CF₂OCH₃ | 90.2 | 99.7 |
| 2 | CF₃CF₂OF(CF₃)COF | (CH₃O)₂SO₂ | CF₃CF₂OCF(CF₃)CF₂OCH₃ | 88.2 | 99.6 |
| 3 | CF₃OCF(CF₃)CF₂OCF(CF₃ )COF | (CH₃O)₂SO₂ | CF₃OCF(CF₃)CF₂OCF(CF₃)CF₂OC H₃ | 85.6 | 99.7 |
| 4 | CF₃CF₂OCF(CF₃)CF₂OCF( CF₃)COF | (CH₃O)₂SO₂ | CF₃CF₂OCF(CF₃)CF₂OCF(CF₃)CF₂ OCH₃ | 87.6 | 99.6 |
| 5 | CF₃OCF(CF₃)COF | (CH₃CH₂O)₂SO₂ | CF₃OCF(CF₃)CF₂OCH₂CH₃ | 88.3 | 99.5 |
| 6 | CF₃OCF(CF₃)CF₂OCF(CF₃ )COF | (CH₃CH₂O)₂SO₂ | CF₃OCF(CF₃)CF₂OCF(CF₃)CF₂OC H₂CH₃ | 85.1 | 99.8 |
| 7 | CF₃CF₂OCF(CF₃)COF | (CH₃CH₂O)₂SO₂ | CF₃CF₂OCF(CF₃)CF₂OCH₂CH₃ | 84.7 | 99.6 |
| 8 | CF₃CF₂OCF(CF₃)CF₂OCF( CF₃)COF | (CH₃CH₂O)₂SO₂ | CF₃CF₂OCF(CF₃)CF₂OCF(CF₃)CF₂ OCH₂CH₃ | 82.6 | 99.7 |

As can be seen from Table 1, by reacting acyl fluoride compounds with dialkyl sulfates, corresponding oxa-hydrofluoroether compounds can be synthesized, and the yield of the target products is > 80%, and the product purity is > 99.5%, showing prospects for large-scale production.

The technical features of the above examples can be arbitrarily combined. For the sake of concise description, not all possible combinations of the technical features in the above examples are described. However, as long as the examples of these technical features do not conflict, they should be considered within the scope of this specification.

The above examples only illustrate several implementations of the present application. Although the descriptions are relatively specific and detailed, they should not be construed as limiting the scope of the present application. It should be noted that, for those of ordinary skill in the art, various modifications and improvements can be made without departing from the concept of the present application, and such modifications and improvements shall fall within the scope of protection of the present application. Therefore, the scope of protection of the present application shall be subject to the appended claims.

## Claims

1. An oxa-hydrofluoroether compound, **characterized in that** the oxa-hydrofluoroether compound has a structure as shown in Formula (1): 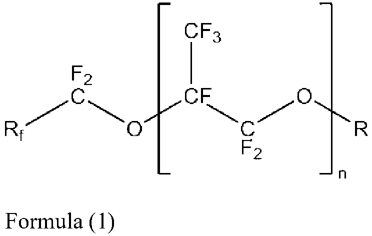 wherein, R_{f} is F or CF₃; R is methyl or ethyl; and n is an integer of 1-3.

2. The oxa-hydrofluoroether compound according to claim 1, **characterized in that** the oxa-hydrofluoroether compound is any one of:
CF3OCF(CF3)CF2OCH3;
CF3OCF(CF3)CF2OCF(CF3)CF2OCH3;
CF3OCF(CF3)CF2OCF(CF3)CF2OCF(CF3)CF2OCH3;
CF3OCF(CF3)CF2OCH2CH3;
CF3OCF(CF3)CF2OCF(CF3)CF2OCH2CH3;
CF3OCF(CF3)CF2OCF(CF3)CF2OCF(CF3)CF2OCH2CH3;
CF3CF2OCF(CF3)CF2OCH3;
CF3CF2OCF(CF3)CF2OCF(CF3)CF2OCH3;
CF3CF2OCF(CF3)CF2OCF(CF3)CF2OCF(CF3)CF2OCH3;
CF3CF2OCF(CF3)CF2OCH2CH3;
CF3CF2OCF(CF3)CF2OCF(CF3)CF2OCH2CH3;
or
CF3CF2OCF(CF3)CF2OCF(CF3)CF2OCF(CF3)CF2OCH2CH3.

3. A synthesis method of an oxa-hydrofluoroether compound, **characterized by** comprising the following steps: reacting an acyl fluoride compound, an alkylating reagent and an alkaline metal fluoride in a polar aprotic solvent to obtain the oxa-hydrofluoroether compound.

4. The synthesis method of an oxa-hydrofluoroether compound according to claim 3, **characterized in that** the acyl fluoride compound has a structure as shown in Formula (2): 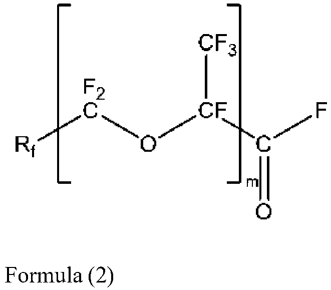 wherein, R_{f} is F or CF₃; and m is an integer of 1-3.

5. The synthesis method of an oxa-hydrofluoroether compound according to claim 4, **characterized in that** the acyl fluoride compound is one or more of:
CF₃OCF(CF₃)COF;
CF₃OCF(CF₃)CF₂OCF(CF₃)COF;
CF₃OCF(CF₃)CF₂OCF(CF₃)CF₂OCF(CF₃)COF;
CF₃CF₂OCF(CF₃)COF;
CF₃CF₂OCF(CF₃)CF₂OCF(CF₃)COF;
or
CF₃CF₂OCF(CF₃)CF₂OCF(CF₃)CF₂OCF(CF₃)COF.

6. The synthesis method of an oxa-hydrofluoroether compound according to claim 3, **characterized in that**: the alkylating reagent is at least one of dialkyl sulfate, dialkyl carbonate, or halogenated hydrocarbon;
the alkaline metal fluoride is at least one of potassium fluoride, sodium fluoride, cesium fluoride, or rubidium fluoride; and
the polar aprotic solvent is at least one of ethylene glycol dimethyl ether, ethylene glycol dibutyl ether, diethylene glycol dimethyl ether, diethylene glycol dibutyl ether, tetraethylene glycol monomethyl ether, tetraethylene glycol dimethyl ether, acetonitrile, N,N-dimethylformamide, N,N-diethylformamide, N-methylpyrrolidone, or dimethyl sulfoxide.

7. The synthesis method of an oxa-hydrofluoroether compound according to claim 3, **characterized in that**: a molar ratio of the acyl fluoride compound to the alkylating reagent is 1:(1-1.5);
a molar ratio of the alkaline metal fluoride to the acyl fluoride compound is 1:(2-10); and
a mass ratio of the acyl fluoride compound to the polar aprotic solvent is 1:(0.8-2).

8. The synthesis method of an oxa-hydrofluoroether compound according to claim 3, **characterized in that** the synthesis method comprises the following steps:
(1) under an protection of nitrogen, mixing the alkaline metal fluoride with the polar aprotic solvent;
(2) after cooling, under the protection of nitrogen, adding the acyl fluoride compound for mixing to obtain a mixture;
(3) dropwise adding the alkylating reagent into the mixture obtained in the step (2), and heating to react after dropwise addition is completed; and
(4) after cooling, adding an alkaline liquor for mixing, followed by standing and distillation to obtain the oxa-hydrofluoroether compound with a purity ≥ 99.5%.

9. The synthesis method of an oxa-hydrofluoroether compound according to claim 8, **characterized in that**: a temperature after cooling in the step (2) is 0-20°C;
before cooling in the step (2), a reaction pressure is adjusted to 0.01-1.0 MPa;
the temperature after heating in the step (3) is 30-120°C, and a reaction time is 2-24 h;
the temperature after cooling in the step (4) is 20-30°C; and
the alkaline liquor in the step (4) is at least one of a sodium hydroxide solution, a potassium hydroxide solution, a sodium carbonate solution, or a potassium carbonate solution; and a mass concentration of the alkaline liquor is 5-50%.

10. Use of the oxa-hydrofluoroether compound of claim 1 or 2 or the oxa-hydrofluoroether compound prepared by the method of any one of claims 3-9 in a preparation of an electronic cleaning agent, an electronic fluorinating liquid, or a solvent.
